# EUROPÄISCHE PATENTSCHRIFT

(11) **EP 2 954 914 B1**
(45) Veröffentlichungstag und Bekanntmachung des Hinweises auf die Patenterteilung: **21.11.2018**
(21) Anmeldenummer: 15167429.8
(22) Anmeldetag: 29.05.2009
(51) Int. Cl.: A61M 5/20, A61M 5/50, A61M 5/32, A61M 5/24, A61M 5/46, A61M 5/31

(54) **INJEKTIONSVORRICHTUNG, INSBESONDERE AUTOINJEKTOR MIT STECHSCHUTZ UND/ODER ÜBERLASTSICHERUNG FÜR EIN PRODUKTBEHÄLTNIS**
INJECTION DEVICE, ESPECIALLY AUTO-INJECTOR, COMPRISING AN ANTI-PRICKING MECHANISM AND/OR OVERLOAD PROTECTION FOR A PRODUCT CONTAINER
DISPOSITIF D'INJECTION, NOTAMMENT AUTO-INJECTEUR COMPRENANT UNE PROTECTION CONTRE LA PIQÛRE INVOLONTAIRE ET/OU UNE SÉCURITÉ CONTRE LA SURCHARGE, POUR UN RÉSERVOIR À PRODUIT

(43) Veröffentlichungstag der Anmeldung: 16.12.2015
(62) Teilanmeldung aus: 09779595.9
(73) Patentinhaber: TecPharma Licensing AG, 3401 Burgdorf (CH)
(72) Erfinder: Streit, Ursina, 3322 Schönbühl (CH); Vogt, Patrick, 8600 Dübendorf (CH)

(56) Entgegenhaltungen:
- EP-A2- 0 956 873
- WO-A2-2007/129324
- US-A- 5 658 259

## Beschreibung

Die Erfindung betrifft eine Injektionsvorrichtung zur Verabreichung eines Produkts, vorzugsweise eines Medikaments. Die Erfindung kann insbesondere in einem Autoinjektor Anwendung finden, wobei sie nicht hierauf beschränkt ist.

Aus dem Stand der Technik sind Autoinjektoren bekannt, die ein selbsttätiges Ausschütten des Produkts erlauben. Das Einstechen der Nadel kann manuell oder automatisch erfolgen. Beim manuellen Einstechen wird die Einstechbewegung der Nadel durch die Hand des Benutzers vermittelt, indem er beispielsweise die Injektionsvorrichtung umgreift und an die Injektionsstelle drückt, wodurch die Nadel eingestochen wird. Bei einem automatischen Einstechen wird die Einstechbewegung der Nadel von einem Antriebsglied, wie z. B. einem Federelement bewirkt, das die Nadel in die Injektionsstelle vortreibt.

Derartige Injektionsvorrichtungen können eine Öffnung aufweisen, aus der die Nadel manuell oder automatisch hervorgeschoben werden kann. Es gibt Systeme, bei denen die Öffnung einen geringfügig größeren Durchmesser als die Nadel aufweist, so dass ein Zugriff mit z. B. einem Finger an die Nadel und somit ein sich versehentlichen Stechen verhindert wird. Diese Ausgestaltungen schränken jedoch die Montierbarkeit der Injektionsvorrichtung ein. Ferner gibt es Systeme, bei denen die Öffnung so groß bemessen ist, dass eine einfache Montierbarkeit der Injektionsvorrichtung möglich ist. Bei diesen Systemen ist die Öffnung in der Regel so groß, dass ein Finger hindurchpasst, wodurch die Gefahr des sich Stechens gegeben ist.

Aus dem Stand der Technik sind ferner Injektionsvorrichtungen bekannt, die ein Produktbehältnis mit dem auszuschüttenden Produkt aufnehmen. An dem distalen Ende des Produktbehältnisses kann eine Nadel angebracht sein oder werden. Es gibt Ausführungen, bei denen das Produktbehältnis an ihrem proximalen, d. h. dem der Nadel entgegengesetzten Ende an der Injektionsvorrichtung befestigt sind. Durch die bei der Verwendung der Injektionsvorrichtung auftretenden Kräfte kann es passieren, dass sich die Befestigung des Produktbehältnisses löst oder das Produktbehältnis im Extremfall bricht, wodurch es aus der Injektionsvorrichtung herausfallen kann. Weitere aus dem Stand der Technik bekannte Injektionsvorrichtungen sind in EP 0 956 873 A2 und US 6099503 offenbart. Die Erfindung kann nicht nur bei den oben beschriebenen, sondern auch bei einer Vielzahl weiterer Injektionsvorrichtungssysteme Anwendung finden.
Der Erfindung liegt die Aufgabe zugrunde, eine Injektionsvorrichtung zu schaffen, bei der die Gefahr des sich Stechens verhindert wird, wobei die Injektionsvorrichtung einfach montierbar bleiben soll. Es ist ferner eine Aufgabe der Erfindung, eine Injektionsvorrichtung bereitzustellen, bei der verhindert wird, dass das Produktbehältnis aus der Vorrichtung herausfallen kann.
Die Aufgaben werden gelöst durch die Merkmale der Ansprüche 1 und 8. Vorteilhafte Weiterentwicklungen ergeben sich aus den abhängigen Ansprüchen.
Die Erfindung geht aus von einer Injektionsvorrichtung zur Verabreichung eines Produkts, bevorzugt eines flüssigen oder fluiden Produkts. Bei dem Produkt kann es sich z. B. um ein Medikament handeln. Das Produkt kann in flüssiger Form in einem Produktbehältnis oder in flüssiger und fester Form in dem Produktbehältnis gelagert werden. Bei letzterem kann es sich um ein so genanntes Zweikammerproduktbehältnis handeln, bei dem ein flüssiger mit einem festen oder pulverförmigen Wirkstoff unmittelbar vor Verabreichung vermischt wird. Die Injektionsvorrichtung kann für ein manuelles oder automatisches Einstechen vorgesehen sein. Bevorzugt ist die Injektionsvorrichtung ein so genannter Autoinjektor.
In einem ersten Aspekt der Erfindung umfasst die Injektionsvorrichtung ein distales Ende und eine Nadel, wobei die Nadel in ihrer Ausgangsposition sich innerhalb der Injektionsvorrichtung befindet und in eine Einstechposition bewegbar ist, in der die Nadel über das distale Ende der Injektionsvorrichtung hervorsteht. Im Auslieferungszustand der Injektionsvorrichtung befindet sich die Nadel in ihrer Ausgangsposition. In der Einstechposition steht die Nadel bevorzugt um ein Maß über das distale Ende der Injektionsvorrichtung hervor, das in etwa der Einstechtiefe der Nadel in die Einstechstelle entspricht. Das distale Ende der Injektionsvorrichtung ist dazu vorgesehen, an bzw. um die Injektionsstelle herum angedrückt zu werden. Die Nadel kann fest mit dem Produktbehältnis verbunden sein, wie z. B. bei der Herstellung des Produktbehältnisses. In dieser Ausführung kann das Produktbehältnis auch als Spritze bezeichnet werden und an seinem proximalen Ende einen Flansch aufweisen, mit dem die Spritze in der Injektionsvorrichtung befestigt werden kann. Alternativ kann die Nadel als separates Teil an dem Produktbehältnis befestigt werden. Das Produktbehältnis kann auch als Karpule oder Ampulle bezeichnet werden. Karpule, Ampulle und Spritze können, aber müssen nicht notwendigerweise am proximalen Ende einen Flansch zur Befestigung aufweisen, der manchmal auch als Fingerflansch bezeichnet wird.

Die Injektionsvorrichtung kann einen Öffnungsbereich aufweisen, der sich in der Ausgangsposition der Nadel distal der Nadel, insbesondere der Nadelspitze, befindet und einen Querschnitt aufweist. Der Öffnungsbereich kann sich in der Ausgangsposition der Nadel zwischen der Nadel bzw. der Nadelspitze und dem distalen Ende der Injektionsvorrichtung erstrecken. Unter der Nadelspitze wird der distale Endbereich der Nadel verstanden. Der Öffnungsbereich kann von einem Gehäuse oder einem Auslöseelement seitlich, d. h. um die Längsachse der Nadel bevorzugt hülsenförmig umgeben sein. Der Öffnungsbereich kann einen Querschnitt, d. h. eine Querschnittsfläche aufweisen, die in etwa normal zur Längsachse der Nadel steht. Der Querschnitt wird begrenzt durch das Element, welches den Querschnitt unmittelbar umgibt. Beispielsweise kann der Öffnungsbereich über seine gesamte axiale Länge einen konstanten oder sich verändernden Querschnitt aufweisen. Bei einem Öffnungsbereich mit mehreren Querschnitten unterschiedlicher Fläche ist bevorzugt der kleinste Querschnitt gemeint, wenn von einem Querschnitt die Rede ist.

Erfindungsgemäß umfasst die Injektionsvorrichtung ein bewegbares Reduzierungsstück. Das Reduzierungsstück kann in den Öffnungsbereich bewegt werden, insbesondere aus einer Position, die nicht im Öffnungsbereich liegt. Zum Beispiel kann die Bewegung entlang, insbesondere parallel zu oder quer, oder kombiniert quer und entlang der Längsachse gerichtet sein. Durch die Bewegung des Reduzierungsstücks in den Öffnungsbereich wird der Querschnitt des Öffnungsbereichs verringert. Das Reduzierungsstück kann an der Stelle, an der es im Öffnungsbereich angeordnet wird, den Querschnitt des Öffnungsbereichs verringern. Zum Beispiel kann das Reduzierungsstück eine Öffnung aufweisen, die einen Querschnitt umfasst, der kleiner ist als der Querschnitt des Öffnungsbereichs an der Stelle, an der das Reduzierungsstück im Öffnungsbereich angeordnet ist oder wird. Bevorzugt ist der Querschnitt des Öffnungsbereichs im Bereich des Reduzierungsstücks so bemessen, dass die Nadel trotz dem im Öffnungsbereich angeordneten Reduzierungsstück durch den Öffnungsbereich bewegbar ist.

Durch die erfindungsgemäßen Merkmale wird der Öffnungsbereich verkleinert, so dass ein Zugriff durch die Öffnung an die Nadel z. B. mit einem Finger nicht mehr möglich ist. Dadurch wird zum einen die Gefahr des sich Stechens als auch der Verunreinigung der Nadel verringert.

In bevorzugten Ausführungen kann die Injektionsvorrichtung ein Abziehelement, insbesondere in der Gestalt einer Kappe, aufweisen, das abnehmbar an der Injektionsvorrichtung, insbesondere an deren distalen Ende angeordnet ist. Das Abziehelement kann im Auslieferungszustand den Zugriff an die Nadel verhindern. Nach Abziehen des Abziehelements, d. h. wenn das Abziehelement von der Vorrichtung entfernt wurde, kann ein Zugriff zum Öffnungsbereich freigegeben sein, wobei der Öffnungsbereich wiederum von dem Reduzierungsstück verengt wird, wodurch der Zugriff in den Öffnungsbereich nicht bis zur Nadel möglich ist.

In bevorzugten Ausführungen ist das Reduzierungsstück aus einer in der Injektionsvorrichtung angeordneten Position, die insbesondere proximal der Nadelspitze liegt, in den Öffnungsbereich bewegbar. Zum Beispiel kann das in der Injektionsvorrichtung aufgenommene Produktbehältnis einen Abschnitt aufweisen, der zur Aufnahme des zu verabreichenden Produkts dient, wobei das Reduzierungsstück aus einer Position, die axial im Bereich des Abschnitts zur Aufnahme des zu verabreichenden Produkts liegt, in den Öffnungsbereich bewegbar ist. Das Produktbehältnis kann einen Kolben aufweisen, der in dem Abschnitt zur Aufnahme des zu verabreichenden Produkts bewegbar ist, insbesondere aus einer proximalen in eine distale Position. Bei dieser Bewegung wird das Produkt verdrängt und durch die Nadel ausgeschüttet. Der Abschnitt zur Aufnahme des zu verabreichenden Produkts liegt proximal des Abschnitts, an dem die Nadel befestigbar oder befestigt ist.

Das Reduzierungsstück kann das Produktbehältnis teilweise, wie z. B. segmentweise, besonders bevorzugt jedoch ringförmig umgeben, z. B. im Bereich des Abschnitts an dem die Nadel befestigt ist. Zum Beispiel kann das Reduzierungsstück einen Durchgang für das Produktbehältnis bilden.

Das Produktbehältnis kann in bevorzugten Ausführungsfonnen im Auslieferungszustand der Injektionsvorrichtung mit einer Nadelschutzkappe versehen sein, die an dem Abschnitt, an dem die Nadel befestigt ist, angeordnet ist. Die Nadelschutzkappe schützt die Nadel vor Verunreinigung und hält sie steril. Beispielsweise kann das Produktbehältnis mit Nadelschutzkappe als fertiges Modul in die Injektionsvorrichtung eingesetzt werden oder eingesetzt sein. Bei der Nadelschutzkappe kann es sich z. B. um eine Kappe aus einem flexiblen, wie z. B. gummiartigen Material, oder um ein festes, wie z. B. aus Hartkunststoff gebildetes Material handeln. Letztere Ausführung wird auch als so genanntes "rigid needle shield" bezeichnet. Bei abgezogenem Abziehelement und abgezogener Nadelschutzkappe liegt die Nadel im Öffnungsbereich der Injektionsvorrichtung frei, wobei durch das Reduzierungsstück das sich Stechen trotzdem verhindert werden kann.

Bevorzugt ist das Reduzierungsstück in einer Position im Öffnungsbereich feststellbar, wie z. B. kraft- und/oder formschlüssig. Beispiel für eine kraftschlüssige Verbindung wäre ein Verklemmen des Reduzierungsstücks im Öffnungsbereich. Beispiel für eine formschlüssige Verbindung wäre beispielsweise eine Rastverbindung, bei der das Reduzierungsstück oder ein Teil des Reduzierungsstücks in ein das Reduzierungsstück umgebende Element einrastet. Das Reduzierungsstück kann hierzu Nocken oder Schnapper aufweisen, die beispielsweise elastisch vorgespannt sind und an der gewünschten axialen Position im Öffnungsbereich in das das Reduzierungsstück umgebende Element einrasten. Zum Beispiel kann das das Reduzierungsstück umgebende Teil eine oder mehrere Vertiefungen aufweisen, in welche das Reduzierungsstück einrastet. Das das Reduzierungsstück umgebende Teil kann z. B. ein Gehäuse, ein Auslöseelement oder eine Nadelschutzhülse sein.

Bevorzugt weist die Öffnung des Reduzierungsstücks einen kleineren Querschnitt auf als der Öffnungsbereich der Injektionsvorrichtung und einen größeren Querschnitt auf als eine an dem Produktbehältnis angeordnete Nadelaschutzkappe und/oder ein Abschnitt zur Aufnahme des zu verabreichenden Produkts. Bei der Montage wird hierdurch vorteilhaft erreicht, dass zumindest die Nadelschutzkappe des Produktbehältnisses durch die Öffnung des Reduzierungsstücks hindurchsteckbar ist, in weiteren Ausführungen auch der Abschnitt zur Aufnahme des zu verabreichenden Produkts.

In bevorzugten Ausführungen ist das Reduzierungsstück relativ zu einem Gehäuse, einer Nadelschutzhülse, einem Auslöseelement und/oder der Nadel bewegbar. Die Nadelschutzhülse kann insbesondere über die in der Einstechposition befindlichen Nadel bewegt werden, um die Nadel nach dem Gebrauch wieder abzudecken. Bevorzugt macht das Reduzierungsstück die Bewegung der Nadelschutzhülse mit, insbesondere dann, wenn das Reduzierungsstück axial fest mit der Nadelschutzhülse verbunden worden ist. Das Auslöseelement dient zum Auslösen der Bewegung der Nadel aus ihrer Ausgangsposition in ihre Einstechposition. Zum Beispiel kann mit dem Auslöseelement ein Energiespeichermittel freigegeben werden, welches die Nadel z. B. mitsamt dem Produktbehältnis verschiebt. In besonders bevorzugten Ausführungen kann die Nadelschutzhülse gleichzeitig die Aufgabe des Auslöseelements übernehmen. Die als Auslöseelement dienende Nadelschutzhülse kann z. B. relativ zu dem Gehäuse in proximale Richtung verschoben werden, beispielsweise dadurch, dass das distale Ende der Nadelschutzhülse an die Injektionsstelle angedrückt wird. Die Nadelschutzhülse oder das Auslöseelement kann distal über das distale Ende des Gehäuses überstehen, wobei beim Andrücken der Injektionsvorrichtung an die Injektionsstelle die Nadelschutzhülse oder das Auslöseelement proximal in das Gehäuse bewegt wird. Beispielsweise kann die Nadelschutzhülse nach Verwendung der Injektionsvorrichtung um ein Maß über das distale Ende des Gehäuses überstehen, das größer ist als das Maß, mit dem die Nadelschutzhülse vor dem Auslösen der Injektion über das distale Ende des Gehäuses übersteht.

Ein weiterer Aspekt, der sowohl für sich alleine weiterverfolgt als auch mit dem ersten Aspekt kombiniert werden kann, betrifft eine Injektionsvorrichtung zur Verabreichung eines Produkts mit dem Produktbehältnis zur Aufnahme des zu verabreichenden Produkts. Das Produktbehältnis weist einen sich verjüngenden Bereich oder Abschnitt auf, der sich insbesondere distal an einen Abschnitt zur Aufnahme des Produkts anschließt. Dieser Abschnitt kann hohlzylindrisch ausgestaltet sein und/oder ein Kolben verschiebbar aufnehmen, mit dem das Produkt aus dem Produktbehältnis verdrängbar ist. Der Kolben kann bevorzugt durch den ganzen Abschnitt zur Aufnahme des Produkts bewegt werden. Das Produktbehältnis kann am proximalen Ende eine nach außen, insbesondere radial sich erstreckende Abragung, wie z. B. einen Flansch aufweisen.

Distal des sich verjüngenden Bereichs des Produktbehältnisses kann ein Befestigungsabschnitt für die Nadel angeordnet sein oder sich anschließen. Der Befestigungsabschnitt kann so ausgestaltet sein, dass eine Nadel an das Produktbehältnis anbringbar und/oder oder von dem Produktbehältnis wieder lösbar ist. Bevorzugt ist die Nadel fest an dem Befestigungsabschnitt angeordnet.

Zumindest unmittelbar vor der Verabreichung liegt die Nadel frei. Im Auslieferungszustand des Produktbehältnisses bzw. der Injektionsvorrichtung kann die Nadel von der Nadelschutzkappe umgeben sein, die bevorzugt an dem Befestigungsabschnitt für die Nadel befestigt ist, insbesondere kraft- und/oder formschlüssig, z. B. durch Aufstecken und/oder mittels Reibschluss. Die Nadelschutzkappe kann vollständig oder zumindest teilweise zum Freigeben der Nadel vom Produktbehältnis entfernbar sein. Die Nadelschutzkappe kann ein so genanntes "rigid needle shield" oder eine einfache Gummikappe sein. Zum Beispiel kann bei einem "rigid needle shield" zum Freigeben der Nadel nur ein Teil der Nadelschutzkappe entfernt werden, wobei ein anderer Teil des "rigid needle shield" am Produktbehältnis insbesondere am Befestigungsabschnitt verbleibt. Der verbleibende und der entfernbare Teil des "rigid needle shield" können z. B. mittels einer stoffschlüssigen oder formschlüssigen Verbindung, insbesondere mit einer Sollbruchstelle verbunden sein, die durch Zerstörung lösbar ist.

Die Injektionsvorrichtung kann ein bewegbares Halteglied aufweisen, das aus einer Position, in der es keine Haltefunktion für das Produktbehältnis erfüllt, in eine Position bewegbar ist, in der es eine Haltefunktion für das Produktbehältnis erfüllt. Bevorzugt ist das Halteglied insbesondere distal vor den sich verjüngenden Bereich, insbesondere eine Schulter des Produktbehältnisses bewegbar. Das Halteglied kann in der Position, in der es die Haltefunktion erfüllt, das Produktbehältnis berühren, muss es aber nicht, so dass ein Abstand zwischen dem sich verjüngenden Bereich und dem Halteglied gemessen in axiale Richtung bestehen kann. Wenn das Halteglied das Produktbehältnis berührt, kann es zur Ausleitung einer auf das Produktbehältnis durch das Abziehen der Nadelabdeckkappe ausgeübten Kraft dienen. Sofern ein Abstand zwischen dem Halteglied und dem sich verjüngenden Bereich besteht, kann das Halteglied dazu dienen, dass es ein Herausfallen des Produktbehältnisses aus der Injektionsvorrichtung verhindert, wenn die Befestigungseinrichtung, mit der das Produktbehältnis in der Injektionsvorrichtung normalerweise aufgenommen ist, versagt. Die Befestigungseinrichtung kann z. B. mittels des am proximalen Ende des Produktbehältnisses angeordneten Flansches ausgestaltet sein, der z. B. an dem proximalen Ende eines Produktbehältnishalters anliegt. Wenn der Flansch, aus welchem Grund auch immer, brechen sollte, verhindert das Halteglied ein Herausfallen des Produktbehältnisses aus der Injektionsvorrichtung.

Das Halteglied kann aus einer ersten Position in die Halteposition verschoben werden. In der ersten Position kann sich das Halteglied seitlich des Produktbehältnisses, insbesondere des Abschnitts zur Aufnahme des Produkts, befinden. In der ersten Position kann das Halteglied so angeordnet sein, dass der sich verjüngende Bereich des Produktbehältnisses an dem Halteglied vorbeiführbar ist. Bevorzugt kann das Halteglied zumindest abschnittsweise an dem Abschnitt zur Aufnahme eines Produkts entlang geführt werden.

Beispielsweise kann mit dem Halteglied bei der Bewegung in die Halteposition eine Radialbewegung in Bezug auf die Längsachse des Produktbehältnisses ausführbar sein. Das Halteglied kann hierzu als Ganzes eine Radialbewegung ausführen oder bevorzugt teilweise. Das Halteglied kann verformt, insbesondere teilweise verformt werden, wenn es in die Halteposition bewegt wird. Die Verformung kann elastisch oder/und plastisch sein. Insbesondere kann das Halteglied zur Längsachse hin geschwenkt werden.

Besonders bevorzugt kann die Injektionsvorrichtung ein Mittel aufweisen, mit dem das Halteglied bei der Bewegung in die Halteposition eine kombinierte Axial- und Radialbewegung ausführen kann. Ein solches Mittel kann z. B. ein Teil eines Getriebes oder einer Getriebefläche oder eine Führungsbahn oder dergleichen sein. Zum Beispiel kann eine Getriebefläche vorgesehen sein, welche in Bezug auf die Längsachse des Produktbehältnisses das Halteglied bei seiner Axialbewegung zur Längsachse hin zwängt. Die Getriebefläche kann das Halteglied zumindest teilweise oder vollständig in die Halteposition zwängen, insbesondere verschieben und/oder verschwenken und/oder verformen.

Das Mittel zur Ablenkung des Halteglieds, wie z. B. die Getriebefläche, kann z. B. an einem Element, welches das Produktbehältnis umgibt, angeordnet oder gebildet sein. Bevorzugt ist dieses Element ein Produktbehältnishalter. Der Produktbehältnishalter kann einen Durchgang für das Produktbehältnis bilden, insbesondere hülsenförmig sein und das Produktbehältnis umgeben. Bevorzugt ist das Halteglied insbesondere radial zwischen dem Produktbehältnis und dem Produktbehältnishalter angeordnet. Somit kann der Produktbehältnishalter sowohl das Halteglied als auch das Produktbehältnis umgeben. Das Halteglied kann bei der Bewegung in die Halteposition relativ zu dem Produktbehältnis und/oder zu dem Produktbehältnishalter bewegbar angeordnet sein. Beispielsweise können Produktbehältnishalter, Halteglied, Produktbehältnis, und insbesondere auch das Gehäuse der Injektionsvorrichtung konzentrisch zueinander angeordnet sein.

Der Produktbehältnishalter kann an seinem distalen Ende eine Öffnung aufweisen, die ein Hindurchstecken eines Teils des Produktbehältnisses erlaubt. Insbesondere ist die Öffnung so groß, dass das Produktbehältnis mit einer an ihm angebrachten Nadelschutzkappe durchgesteckt werden kann. Die Öffnung kann aber muss nicht so groß sein, dass der Abschnitt zur Aufnahme des zu verabreichenden Produkts durch die Öffnung hindurchpassen würde. Bevorzugt ist das Mittel zur Ablenkung des Halteglieds am distalen Ende des Produktbehältnishalters angeordnet, das insbesondere über den Umfang des Produktbehältnishalters gebildet ist. Das Mittel zur Ablenkung kann eine zur Längsachse geneigte Fläche aufweisen.

Das Halteglied kann das Produktbehältnis über dessen Umfang umgeben. Insbesondere kann das Halteglied mehrere Zungen umfassen, die durch Materialverformung vor den sich verjüngenden Bereich des Produktbehältnisses bewegbar sind. In bevorzugten Ausführungsformen können die Zungen gemeint sein, wenn von einem Halteglied die Rede ist, das vor den verjüngenden Bereich des Produktbehältnisses bewegbar ist. Das bedeutet, dass nicht das ganze Halteglied vor den sich verjüngenden Bereich, sondern lediglich ein Teil des Halteglieds vor dem sich verjüngenden Bereich bewegbar sein braucht. Beispielsweise kann das Halteglied eine ringförmige Basis aufweisen, von der sich die mindestens eine Zunge erstreckt, insbesondere in Richtung der Längsachse.

Besonders bevorzugt wird das Halteglied in der Position vor dem sich verjüngenden Bereich des Produktbehältnisses verrastet oder verrastbar ist gegen eine weitere Bewegung relativ zu dem Produktbehältnis. Hierzu kann beispielsweise die mindestens eine Zunge oder die ringförmige Basis oder ein anderes Teil des Halteglieds ein Rastelement aufweisen, das bevorzugt in das das Rasteelement umgebende Teil insbesondere den Produktbehältnishalter eingreift. Zum Beispiel kann das Rastelement der Zunge in das Ablenkmittel für das Halteglied bzw. die Zunge eingreifen.

Die Injektionsvorrichtung kann mindestens ein Eingriffsglied aufweisen, das kraft- oder/und formschlüssig mit dem wenigstens einen aus Reduzierungsstück und Halteglied gekoppelt ist, wodurch bei einer Axialbewegung des mindestens einen Eingriffsglieds das Reduzierungsstück oder/und das Halteglied mitnehmbar ist. Bevorzugt kann das mindestens eine Eingriffsglied in distale Richtung bewegt werden. Bei dieser Bewegung kann es das Eingriffsglied zumindest abschnittsweise mitnehmen. Hierzu kann das Eingriffsglied Laschen aufweisen, die sich bevorzugt von der ringförmigen Basis erstrecken, insbesondere in Längsrichtung und bevorzugt in die gleiche Richtung wie die mindestens eine Zunge. In bevorzugten Ausführungen kann das mindestens eine Eingriffsglied in distale Richtung bewegt werden und nimmt bei dieser Bewegung das Halteglied zumindest abschnittsweise mit, wobei bei der Mitnahme keine Relativbewegung zwischen Halteglied und dem mindestens einen Eingriffsglied stattfindet. Hierzu kann das mindestens eine Eingriffsglied in die mindestens eine Lasche des Halteglieds eingreifen. Das Halteglied und das mindestens eine Eingriffsglied sind so lange miteinander bewegbar, bis das Eingriffsglied vor das Produktbehältnis bewegt ist. Eine weitere Bewegung des mindestens einen Eingriffsglieds ist bevorzugt relativ zum Halteglied möglich.

Das Reduzierungsstück kann sich bei der Bewegung des Halteglieds mit dem Halteglied mitbewegen, beispielsweise indem es sich an dem Halteglied abstützt. Alternativ oder zusätzlich kann sich das Reduzierungsstück mit dem wenigstens einen Eingriffsglied mitbewegen, beispielsweise indem das Eingriffsglied in das Reduzierungsstück eingreift. Bevorzugt kann bei der Bewegung des wenigstens einen Eingriffsglieds in distale Richtung die Nadelschutzkappe von dem Produktbehältnis abgezogen werden, wobei dies vor, nach oder während der Mitnahme des Reduzierungsstücks oder/und des Halteglieds geschehen kann.

Das wenigstens eine Eingriffsglied kann an einem Abziehelement, insbesondere einer Kappe, gebildet sein, insbesondere an einem oder mehreren sich in Längsrichtung erstreckenden Armen, die das mindestens eine Eingriffsglied quer zur Längsachse federnd lagern. Beim Abziehen des Abziehelements von der Injektionsvorrichtung können somit folgende Schritte ausgeführt werden:
- Abziehen der Nadelschutzkappe oder/und
- Mitnahme des Halteglieds, so dass dessen Zungen sich vor die Schulter des Produktbehältnisses bewegen oder/und
- Mitnahme des Reduzierungsstücks in eine Position, in der es den Querschnitt des Öffnungsbereich verringert und somit den Zugriff auf die Nadel verhindert.

Allgemein kann die Injektionsvorrichtung wenigstens eines der folgenden Merkmale umfassen:
- eine als Auslöseelement zum Auslösen einer Einstechbewegung einer Nadel dienenden Nadelschutzhülse, wobei die Nadelschutzhülse zum Auslösen der Einstechbewegung relativ zu dem Gehäuse der Injektionsvorrichtung in proximale Richtung verschiebbar ist und in distale Richtung über die in der Einstechposition befindliche Nadel verschiebbar ist,
- ein Elastizitätsmittel, insbesondere eine Feder, gegen welche das Auslöseelement zum Auslösen verschiebbar ist und mit der das Auslöseelement in distale Richtung über die in der Einstechposition befindliche Nadel verschiebbar ist,
- ein Abtriebsglied, welches von einem Antriebsglied, insbesondere einer Vortriebsfeder, in distale Richtung verschiebbar ist, um das Produktbehältnis für eine Einstechbewegung der Nadel zu verschieben und/oder einen Kolben in dem Produktbehältnis für eine Produktausschüttung zu bewegen,
- ein Sperrglied, welches wahlweise in einem Eingriff mit dem Abtriebsglied oder dem Auslöseelement bringbar ist, um eine Axialbewegung desjenigen Teils zu verhindern, mit dem das Sperrglied in einem Eingriff ist.

Die Erfindung wurde anhand mehrerer Ausführungen beschrieben. Im Folgenden wird die Erfindung anhand eines bevorzugten Beispiels erläutert. Die hierbei offenbarten Merkmale bilden je einzeln und in Kombination den Gegenstand der Erfindung vorteilhaft weiter. Es zeigen:
- Figur 1: zwei Baugruppen der Injektionsvorrichtung vor einer Endmontage,
- Figur 2a: die Einzelteile der Injektionsvorrichtung in einer perspektivischen Ansicht,
- Figur 2b: eine vergrößerte Ansicht von drei Teilen der Ansicht aus Figur 2a,
- Figur 2c: eine Anordnung der drei Teile aus Figur 2b wie in einem Auslieferungszustand,
- Figuren 3a und 3b: Längsschnitte der Injektionsvorrichtung mit aufgesetztem Abziehelement, wobei Figur 3b in Bezug auf Figur 3a eine um 90° um die Längsachse gedrehte Ansicht ist,
- Figuren 4a und 4b: Längsschnitte der Injektionsvorrichtung in einem Ausgangszustand, von der das Abziehelement entfernt wurde, wobei Figur 4b in Bezug auf Figur 4a eine um 90° um die Längsachse gedrehte Ansicht ist,
- Figuren 5a und 5b: Längsschnitte der Injektionsvorrichtung in einem ausgelösten Zustand, wobei Figur 5b in Bezug auf Figur 5a eine um 90° um die Längsachse gedrehte Ansicht ist,
- Figuren 6a und 6b: Längsschnitte der Injektionsvorrichtung in einem Zustand nach der Einstechsequenz und vor der Ausschüttsequenz, wobei Figur 6b in Bezug auf Figur 6a eine um 90° um die Längsachse gedrehte Ansicht ist,
- Figuren 7a und 7b: Längsschnitte der Injektionsvorrichtung nach einer Ausschüttsequenz, wobei Figur 7b in Bezug auf Figur 7a eine um 90° um die Längsachse gedrehte Ansicht ist, und
- Figuren 8a und 8b: Längsschnitte der Injektionsvorrichtung mit einer Nadelschutzhülse in ihrer Nadelschutzposition, wobei Figur 8b in Bezug auf Figur 8a eine um 90° um die Längsachse gedrehte Ansicht ist.

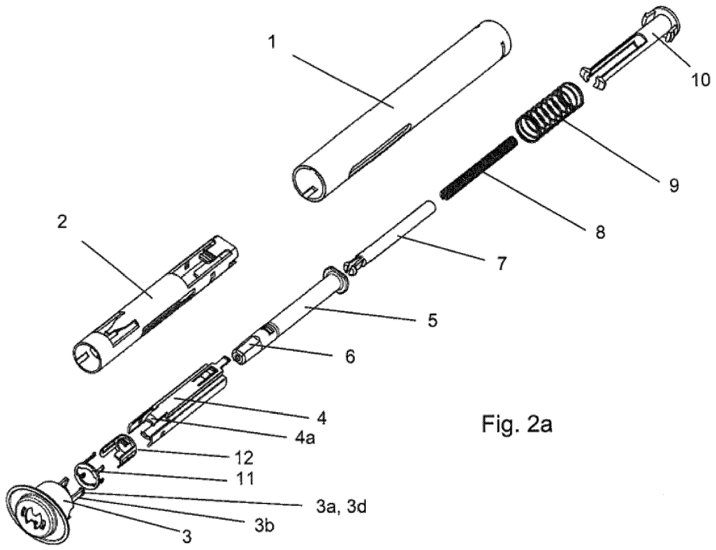

Zunächst werden mit Bezug auf die Figuren 2a bis 2c die Einzelteile einer Injektionsvorrichtung, die im Speziellen einen Autoinjektor bilden, beschrieben. Die Injektionsvorrichtung umfasst ein hülsenförmiges Gehäuse 1, eine Nadelschutzhülse 2, die in dem Gehäuse 1 längsverschiebbar aufgenommen ist und gleichzeitig als Auslöseelement dient, ein Produktbehältnis 5 mit einer daran lösbar befestigten Nadelschutzkappe 6, einen Produktbehältnishalter 4, der das Produktbehältnis 5 aufnimmt und Mittel 4a zur Ablenkung aufweist, ein Abziehelement 3, das Eingriffsglieder 3a, 3d aufweist, ein Antriebsglied 8 in der Gestalt einer als Druckfeder wirkenden Wendelfeder, die die Energie für Einstech- und Ausschüttsequenzen liefert, ein Abtriebsglied 7, welches auf das Produktbehältnis 5 wirkt, ein Halteelement 10, das das Antriebsglied 8 bis zur Auslösung der Injektionsvorrichtung in einem gespannten Zustand hält und das axial fest mit dem Gehäuse 1 verbunden, insbesondere verschnappt ist, und ein Federelement 9, das die Energie für die Verschiebung der Nadelschutzhülse 2 in die Nadelschutzposition liefert. Außerdem umfasst die Injektionsvorrichtung ein Halteglied 12, das eine einfache Montage erlaubt und dennoch ein Herausfallen des Produktbehältnisses aus der Injektionsvorrichtung verhindern kann, und ein Reduzierungsstück 11, das als Zugriffsschutz dient, um ein sich Stechen an der Vorrichtung zu verhindern. Die hier gezeigte bevorzugte Ausführung beinhaltet sowohl das Halteglied 12 als auch das Reduzierungsstück 11. Grundsätzlich kann die Injektionsvorrichtung nur mit einem der beiden genannten Teile ausgestattet sein.

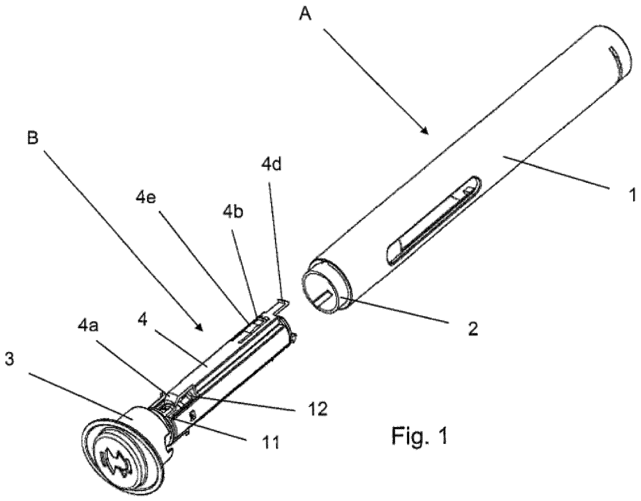

Figur 1 zeigt den Autoinjektor vor einer Endmontage. Bei der Endmontage liegt der Autoinjektor in zwei Baugruppen vor, nämlich einer Baugruppe A, welche im Wesentlichen das Gehäuse, die Nadelschutzhülse 2 und die Antriebseinheit bereits in einem vorgespannten Zustand umfasst, und einer Baugruppe B, welche im Wesentlichen das Produktbehältnis 5, den Produktbehältnishalter 4, das Abziehelement 3 und wenigstens eines aus Halteglied 12 und Reduzierungsstück 11 umfasst. Diese Baugruppenaufteilung hat den Vorteil, dass die Vormontage der Baugruppen an einem anderen Ort erfolgen kann als die Endmontage bzw. Integration des Produktbehältnisses. Beispielsweise können die Baugruppe A und die Baugruppe B ohne das Produktbehältnis von einem ersten Standort an einen zweiten Standort geliefert werden, wobei am zweiten Standort das Produktbehältnis 5 mitsamt Nadelschutzkappe 6 über eine Öffnung am proximalen Ende des Produktbehältnishalters 4 in die Kombination aus Produktbehältnishalter 4 und Abziehelement 3 mit der Nadelschutzkappe 6 voran in den Produktbehältnishalter 4 eingeführt bzw. eingesteckt wird. Das Halteglied 12 und das Reduzierungsstück 11 weisen jeweils eine Öffnung 11d; 12d auf, welche groß genug ist, die Nadelschutzkappe 6 des Produktbehältnisses 5 durch die Öffnungen 11d; 12d zu stecken. Halteglied 12 und Reduzierungsstück 11 sind jeweils in ersten Positionen angeordnet und werden jeweils erst in zweite Positionen verschoben, wenn das Abziehelement 3 von der Injektionsvorrichtung entfernt wird. Somit ist eine einfache Integration des Produktbehältnisses 5 einschließlich Nadelschutzkappe 6 in die restlichen Teile der Baugruppe B möglich.
Die fertig montierte Baugruppe B kann nun über eine Öffnung am distalen Ende des Auslöseelements 2 in die Baugruppe A eingeführt werden, wo sie mit einem Befestigungsglied 4b des Produktbehältnishalters 4, das in diesem Beispiel als Schnapper gebildet ist, axial fest, aber lösbar mit dem Gehäuse 1 befestigt wird. Ein weiterer Vorteil der Baugruppenaufteilung ist, dass eine Vielzahl von Baugruppen A, B vom ersten Standort an den zweiten Standort geliefert werden können und erst am zweiten Standort entschieden wird, mit welchem Medikament die Injektionsvorrichtung ausgestattet werden soll. Dies erhöht die flexible Verwendbarkeit der Injektionsvorrichtung.

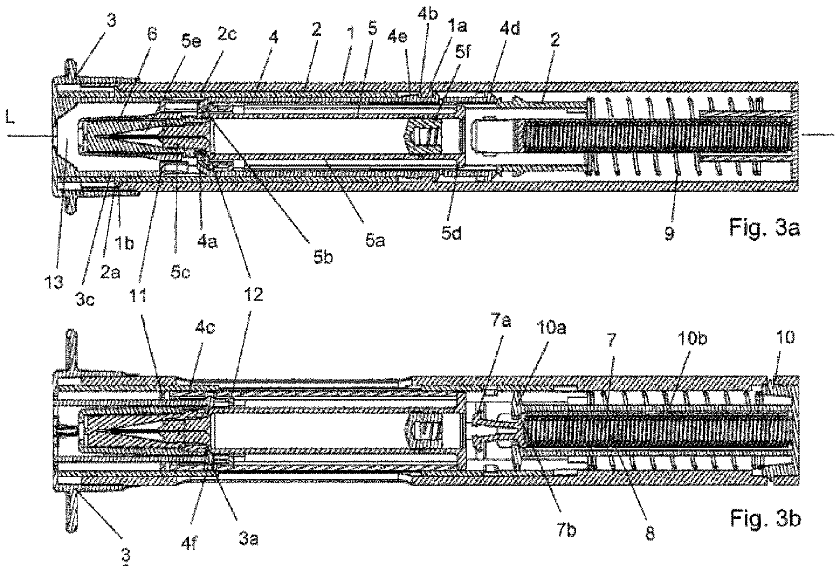

Figuren 3a und 3b zeigen die Injektionsvorrichtung in einem fertig montierten Auslieferungszustand. Ergänzend wird auf die Darstellung der Einzelteile in den Figuren 2a bis 2c verwiesen. In dem Gehäuse 1 ist das Produktbehältnis 5 verschiebbar gelagert. Das Produktbehältnis 5 ist in dem Produktbehältnishalter 4 aufgenommen, so dass es insbesondere nicht in distale Richtung relativ zum Produktbehältnishalter 4 bewegbar ist. Dies wird bewirkt durch einen an dem proximalen Ende des Produktbehältnisses 5 gebildeten Fingerflansch 5d, der radial nach außen ragt und an die proximale Stirnseite des Produktbehältnishalters 4 angreift. Der Reservoirteil 5a des Produktbehältnisses 5 nimmt das zu verabreichende Produkt auf. In dem hohlzylindrischen Reservoirteil 5a ist ein Kolben 5f aufgenommen, der dichtend an der Innenwand des Reservoirteils 5a anliegt und relativ zu dem Reservoirteil 5a für eine Produktausschüttung in Richtung Nadel 5e verschiebbar ist. Die Nadel 5e ist an einem Befestigungsabschnitt 5c des Produktbehältnisses 5 unlösbar befestigt. Der Befestigungsabschnitt 5c schließt sich distal an den Reservoirteil 5a an. Der Reservoirteil 5a geht mittels einer Schulter 5b in den Befestigungsabschnitt über. Die Schulter 5b bildet somit einen sich verjüngenden Bereich, vor oder an dem im späteren Verlauf zumindest ein Teil des Halteglieds 12 bewegt wird. Um die Nadel 5e vor Verschmutzung zu schützen und steril zu halten, ist am Befestigungsabschnitt 5c und über der Nadel 5e die Nadelschutzkappe 6 angeordnet. Die Nadelschutzkappe 6 ist kraft- und/oder formschlüssig an dem Befestigungsabschnitt 5c des Produktbehältnisses 5 befestigt. Zwischen der Schulter 5b des Produktbehältnisses 5 und dem proximalen Ende bzw. der proximalen Stirnseite der Nadelschutzkappe 6 besteht eine Lücke, die zumindest so groß ist, dass zumindest eines oder beide aus den Eingriffsgliedern 3a, 3d und einen Teil des Halteglieds 12 in die Lücke eingreifen können.

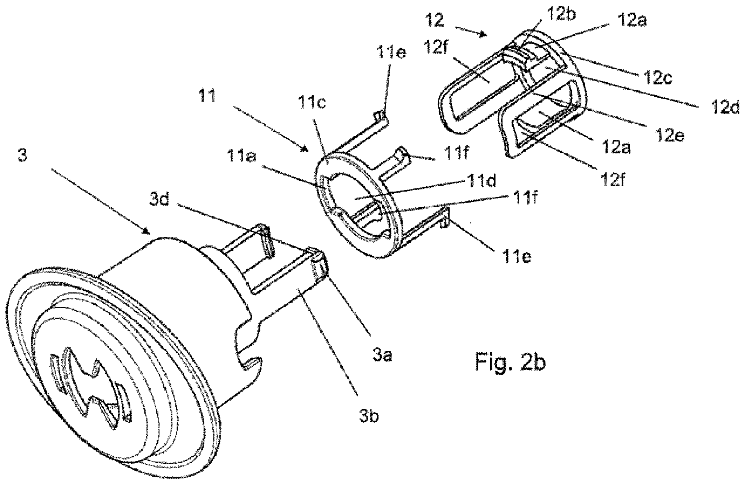

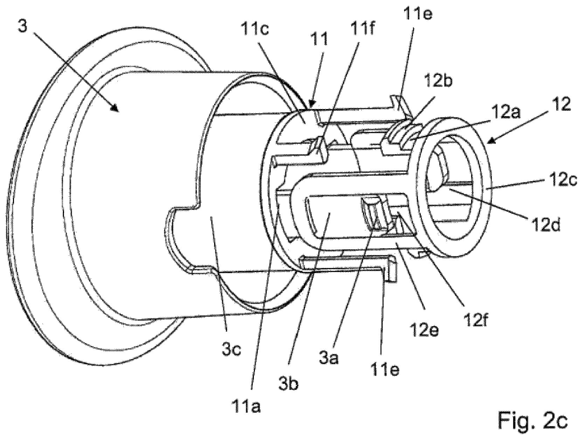

Das Halteglied 12 und das Reduzierungsstück 11 werden insbesondere in den Figuren 2b und 2c detailliert gezeigt. Das Reduzierungsstück 11 weist eine ringförmige Basis 11c auf. Die ringförmige Basis 11c umfasst einen Durchgang oder eine Öffnung 11d, die so bemessen ist, dass die Nadelschutzkappe 6 zumindest teilweise, vorzugsweise vollständig durchgesteckt bzw. durchbewegt werden kann. Andererseits ist die Öffnung 11d so bemessen, dass vorzugsweise ein menschlicher Finger nicht hindurchpasst. Der Querschnitt der Öffnung 11d ist kleiner als der Querschnitt des Öffnungsbereichs 13 (Figur 3a), wobei durch das Anordnen des Reduzierungsstücks 11 im Öffnungsbereich 13 der Querschnitt des Öffnungsbereichs reduziert bzw. verringert wird. Die ringförmige Basis 11c weist ferner Ausnehmungen 11a auf, die insbesondere als Durchgang und seitlich der Öffnung 11d gebildet sind. Die Ausnehmungen 11a können von der Öffnung 11d getrennt oder - wie hier gezeigt - mit der Öffnung 11d verbunden sein. Die Ausnehmungen 11a dienen als Durchgang für jeweils einen Arm 3b, der an dem mindestens einen Eingriffsglied 3a, 3d gebildet ist.

Das Reduzierungsstück 11 weist mehrere in proximale Richtung von der ringförmigen Basis 11c sich erstreckende Abragungen auf, die Anschläge oder Rastelemente 11e, 11f für gleiche oder unterschiedliche Zwecke bilden. Hierzu können die Abragungen gleich oder unterschiedlich lang ausgebildet sein.

Das Rastelement 11e kann aber muss nicht für einen Anschlag an das Halteglied 12, insbesondere an deren ringförmige Basis 12c dienen. Der Anschlag bewirkt, dass bei einer Bewegung des Halteglieds 12 in distale Richtung das Reduzierungsstück 11 von dem Halteglied 12 mitgenommen wird. Sofern das Rastelement 11e nicht als Anschlag dient, kann das Reduzierungsstück 11 von dem mindestens einen Eingriffsglied 3a, 3d mitgenommen werden.

Die Rastelemente 11e, 11f verrasten axialfest mit der Nadelschutzhülse 2, wenn sich das Reduzierungsstück 11 in seiner zweiten Position, d. h. im Öffnungsbereich 13 befindet. Das Reduzierungsstück 11 macht aufgrund der axialfesten Verrastung mit der Nadelschutzhülse 2 die Bewegungen der Nadelschutzhülse 2 mit. In dem gezeigten Beispiel weist das Reduzierungsstück 11 zwei Rastelemente 11e und zwei Rastelemente 11f auf, wobei die Rastelemente 11e an Abragungen gebildet sind, die länger sind als die Abragungen, an denen die Rastelemente 11f gebildet sind. Zum Beispiel können die Rastelemente 11e für eine Sperrung der Axialbewegung in distale Richtung und die Rastelemente 11f für eine Sperrung der Bewegung des Reduzierungsstücks 11 relativ zur Nadelschutzhülse 2 in proximale Richtung dienen. Somit wird, wie allgemein bevorzugt, erreicht, dass das Reduzierungsstück 11 relativ zur Nadelschutzhülse 2 in beide Axialrichtungen nicht bewegbar mit der Nadelschutzhülse 2 verbunden ist.

Das Halteglied 12 weist eine ringförmige Basis 12c auf, von der in distale Richtung Zungen 12a und Laschen 12e abragen. Die Zungen 12a sind flexibel, insbesondere elastisch oder plastisch zur Mittel- oder Längsachse des Halteglieds 12 hin verformbar, insbesondere biegbar. Die Zungen 12a weisen insbesondere an ihren distalen Enden jeweils ein Rastglied 12b auf, welches im späteren Verlauf mit dem Ablenkmittel 4a verrasten kann.

Die Laschen 12e weisen jeweils eine Nut 12f auf, die sich in Längsrichtung erstreckt. Die Nuten dienen für einen Eingriff für Eingriffsglieder 3a, alternativ 3d des Abziehelements 3. Die Nuten 12f sind durchgängig, könnten allerdings auch Sacknuten sein. Die Arme 3b greifen durch die Ausnehmungen 11a des Reduzierungsstücks 11 hindurch, so dass das mindestens eine Eingriffsglied 3a in die Nut 12f eingreifen kann.

Die ringförmige Basis 12c bildet einen Durchgang bzw. eine Öffnung 12d, die groß genug ist, dass die Nadelschutzkappe 6 durchgesteckt oder hindurchbewegt werden kann. Bevorzugt versperren die Rastglieder 12b in ihrem unverformten Zustand den Durchgang für die Nadelschutzkappe 6 nicht.

Das Ablenkmittel 4a ist federnd an dem Produktbehältnishalter 4 angeordnet, nämlich mittels einem Arm 4g. Diese Anordnung hat Montagevorteile. Wie aus Figur 1 erkennbar ist, wird das Produktbehältnis 5 in den in Umfangsrichtung freiliegenden Produktbehältnishalter 4 eingesteckt. Da der Produktbehältnishalter 4 vor der Endmontage in Umfangsrichtung freiliegt, kann das Ablenkmittel 4a nach außen wegfedern und somit die Nadelschutzkappe 6 durchlassen, ohne dass eine erhöhte Montagekraft notwendig ist oder die Gefahr des Verklemmens besteht. Wegen der nachträglichen Querschnittsverringerung durch das Halteglied 12 ist es allerdings möglich, dass die Ablenkmittel 4a nicht nach außen wegfedern brauchen, da der Durchgang für die Nadelschutzhülse 6 zunächst groß genug ist. Daher braucht das Ablenkmittel nicht zwingend federnd sondern kann auch starr angeordnet sein. Im endmontierten, wie z. B. in den Figuren 3a und 3b gezeigten Zustand, kann das Ablenkmittel 4a nicht nach außen wegfedern, da der Produktbehältnishalter 4 von der Nadelschutzhülse 2 umgeben wird, wobei die Nadelschutzhülse 2 einen Halteabschnitt 2c an ihrer Innenseite bildet, der das Ablenkmittel 4a an einer Bewegung radial nach außen hindert.

Der Produktbehältnishalter 4 weist ein nach außen gerichtetes Befestigungsglied 4b auf, in der Gestalt einer Aussparung, wobei das Befestigungsglied 4b in eine am inneren Umfang des Gehäuses gebildete Abragung 1a eingreift und somit eine formschlüssige Verriegelung bildet. Diese Verriegelung ist jedoch während der Verwendung der Injektionsvorrichtung lösbar. Die Verriegelung bewirkt, dass der Produktbehältnishalter 4 axial fest mit dem Gehäuse 1 gekoppelt ist, wodurch auch das Produktbehältnis 5 relativ zum Produktbehältnishalter 4 nicht in distale Richtung bewegbar ist aufgrund des Eingriffs des Fingerflanschs 5d an die proximale Stirnseite des Produktbehältnishalters 5.

Die Nadelschutzhülse 2 ist am inneren Umfang des Gehäuses 1 geführt. Insbesondere befindet sich die Nadelschutzhülse 2 radial zwischen dem Produktbehältnishalter 4 und dem Gehäuse 1. Grundsätzlich ist die Nadelschutzhülse 2 relativ zum Gehäuse 1 verschiebbar, wobei die Nadelschutzhülse 2 im Auslieferungszustand relativ zum Gehäuse 1 axial fest ist. Die Nadelschutzhülse 2 weist hierzu ein Eingriffsglied 2a auf, welches formschlüssig in ein Eingriffsgegenglied 1b an der Innenseite des Gehäuses 1 eingreift. Das Eingriffsglied 2a ist federnd gelagert und kann quer zur Längsachse der Injektionsvorrichtung bewegt werden. Die federnde Anordnung wird durch einen Arm, an dessen Ende das Eingriffsglied 2a geformt ist, gebildet.

Das Abziehelement 3 verschließt das distale Ende der Injektionsvorrichtung zumindest teilweise. Insbesondere verhindert das Abziehelement 3 einen Zugriff auf die Nadelschutzhülse 2, die über das distale Ende des Gehäuses 1 übersteht. Das Abziehelement 3 ist kraftschlüssig an dem Gehäuse 1 befestigt und zwar mittels einem hülsenartigen Fortsatz, der das distale Ende des Gehäuses 1 umgibt. Alternativ oder zusätzlich kann das Abziehelement 3 formschlüssig an dem distalen Ende der Injektionsvorrichtung, wie z. B. an dem Gehäuse 1 oder der Nadelschutzhülse 2 oder über den Eingriff des Eingriffsglieds 3a in den Produktbehältnishalter 4, insbesondere in dessen Aussparung 4f befestigt sein. Das Abziehelement 3 weist ferner einen hülsenförmigen Halteabschnitt 3c auf, der sich im Auslieferungszustand der Injektionsvorrichtung axial auf Höhe des Eingriffsglieds 2a am inneren Umfang der Nadelschutzhülse 2 befindet, um eine Bewegung des Eingriffsglieds 2a nach innen zu verhindern, und somit sicherstellt, dass eine Bewegung der Nadelschutzhülse 2 relativ zum Gehäuse 1 im Auslieferungszustand, d. h. mit aufgesetztem Abziehelement 3 verhindert wird. Somit kann auf sichere Weise verhindert werden, dass sich die Nadelschutzhülse 2 relativ zum Gehäuse 1 bewegt, wie z. B. wenn die Injektionsvorrichtung herunterfällt und sich die Nadelschutzhülse 2 aufgrund Massenträgheit relativ zum Gehäuse 1 bewegen würde. Das Eingriffsglied 2a dient somit vornehmlich einer Transportsicherung. Das Abziehelement 3 bildet einen Ringspalt, nämlich zwischen der äußeren Hülse, die am Umfang des Gehäuses 1 befestigt ist und der inneren Hülse, die den Halteabschnitt 3c aufweist. Im aufgesetzten Zustand des Abziehelements befinden sich ein Teil des Gehäuses 1 und ein Teil der Nadelschutzhülse 2 im Ringspalt, vorzugsweise auch das Eingriffsglied 2a und das von dem Gehäuse gebildete Eingriffsgegenglied 1b. Der Halteabschnitt 3c, insbesondere dessen proximales Ende, bildet außerdem eine Montagehilfe für die Vormontage der Baugruppe B, indem sich zum Beispiel das Reduzierungsstück 11, insbesondere die ringförmige Basis 11e an dem proximalen Ende des Abziehelements 3 bzw. des Halteabschnitts 3c und optional wenigstens eines aus Halteglied 12 und Produktbehältnishalter 4 am Reduzierungsstück 11 abstützen können.

An dem Abziehelement 3 ist mindestens ein Eingriffsglied 3a, 3d gebildet, das über den Arm 3b federnd quer zur Längsachse der Injektionsvorrichtung bewegbar ist. Während der Vormontage der Baugruppe B und auch im Auslieferungszustand der Injektionsvorrichtung befindet sich die an dem Produktbehältnishalter 4 gebildete Aussparung 4f in Längsrichtung auf Höhe des Eingriffsglieds 3a, so dass das Eingriffsglied 3a radial nach außen in die Aussparung federn kann. Beim Einsetzen des Produktbehältnisses 5 in die Kombination aus Produktbehältnishalter 4 und Abziehelement 3 während der Montage der Baugruppe B (Figur 1) federt das Eingriffsglied 3a in die Aussparung 4f, wenn die Nadelschutzkappe 6 das Eingriffsglied 3a passiert. Ferner federt das Eingriffsglied 3a in die Aussparung 4f, wenn das Produktbehältnis 5 vollständig in den Produktbehältnishalter 4 eingesetzt ist, da in diesem Fall das Eingriffsglied 3a am distalen Endbereich, insbesondere seitlich an dem Reservoirteil 5a des Produktbehältnisses 5 anliegt. Alternativ kann das Eingriffsglied 3a oder ein anderes Eingriffsglied 3d in diesem Zustand in die Lücke zwischen Nadelschutzkappe 6 und Schulter 5b eingreifen. Das Eingriffsglied 3a ragt in Bezug auf den äußeren Umfang des hülsenförmigen Abschnitts, der den Halteabschnitt 3c bildet, radial nach außen ab und bildet dadurch eine Art Getriebefläche, deren Funktion später beschrieben wird.

Das Eingriffsglied 3d ragt in Bezug auf den inneren Umfang des hülsenförmigen Abschnitts, der den Halteabschnitt 3c bildet, nach innen und ist bevorzugt hakenförmig ist und so dimensioniert, dass es im Verlauf der Verwendung der Injektionsvorrichtung in die Lücke zwischen Nadelschutzkappe 6 und Schulter 5b eingreifen kann.

Da in den gezeigten Ausführungen die Eingriffsglieder 3a, 3d in etwa auf der gleichen Axialposition angeordnet sind, können sie ein gemeinsames Eingriffsglied bilden. Grundsätzlich können die Eingriffsglieder 3a und 3d auf unterschiedlichen Axialposition angeordnet sein.

Das Abziehelement 3 weist schließlich noch eine radial nach außen gerichtete Abragung auf, die es dem Verwender der Injektionsvorrichtung erleichtert, das Abziehelement 3 zu greifen und eine Axialkraft auf das Abziehelement 3 aufzubringen.

Bei vollständig in den Produktbehältnishalter 4 eingesetztem Produktbehältnis 5 kann die Abragung 5d in Längsrichtung an dem proximalen Ende des Produktbehältnishalters 4 anliegen.

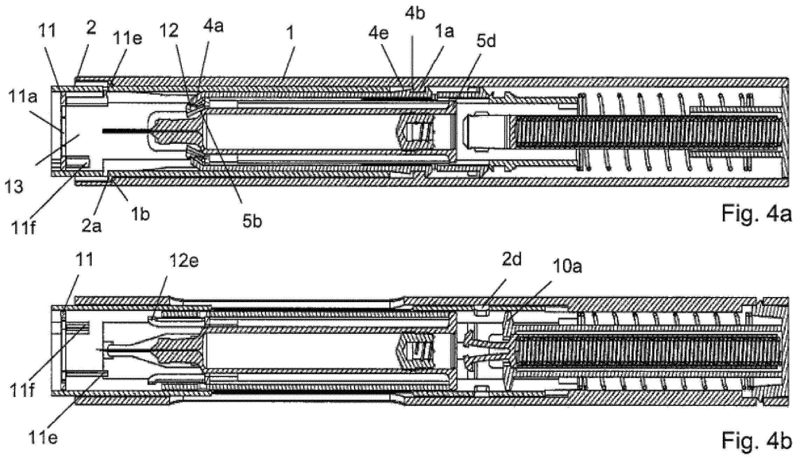

Das Federelement 9 stützt sich mit seinem proximalen Ende an dem Halteelement 10 und somit axial fest zum Gehäuse 1 und mit seinem distalen Ende 9 am proximalen Ende der Nadelschutzhülse 2 ab. Das Federelement 9 ist vorgespannt und beaufschlagt die Nadelschutzhülse 2 mit einer in distale Richtung wirkenden Kraft, wobei die Nadelschutzhülse 2 sowohl in der Ausgangsposition als auch unmittelbar nach dem Abziehen des Abziehelements 3 (Figuren 4a und 4b) für eine Bewegung in distale Richtung gesperrt ist.

Das Halteelement 10 verschließt das proximale Ende des Gehäuses 1 und ist mit dem Gehäuse 1 dreh- und axialfest verbunden, insbesondere verschnappt. Das Halteelement 10 könnte genauso gut einteilig mit dem Gehäuse 1 gebildet sein, wobei es vorteilhaft ist, Halteelement 10 und Gehäuse 1 mehrteilig zu gestalten, da hierdurch die Herstellbarkeit der Einzelteile und die Montage der Injektionsvorrichtung erleichtert wird.

Das Halteelement 10 weist mindestens einen, in diesem Beispiel zwei federnde Arme 10b auf, die sich in Längsrichtung der Injektionsvorrichtung erstrecken und an ihren distalen Enden ein Sperrglied 10a bilden. Das Sperrglied 10a ist quer zur Längsachse der Injektionsvorrichtung bewegbar. Das Sperrglied 10a bildet in Bezug auf den Arm 10b eine nach innen gerichtete und eine nach außen gerichtete Abragung. Die nach innen gerichtete Abragung greift an eine von dem Abtriebsglied 7 gebildete Schulter 7b formschlüssig ein und verhindert dadurch, dass sich das Abtriebsglied 7 in distale Richtung bewegt. Die nach außen gerichtete Abragung des Sperrglieds 10a liegt an einer nach innen weisenden Fläche der Nadelschutzhülse 2 an, so dass das Sperrglied in dem Eingriff mit der Schulter 7b gehalten und eine Bewegung des Sperrglieds 10a radial nach außen verhindert wird. Die Arme 10b sind mit ihren proximalen Enden an einem hülsenförmigen Abschnitt des Halteglieds 10 gebildet.

Der Arm 10b erstreckt sich zusammen mit dem hülsenförmigen Abschnitt des Halteglieds 10 über die gesamte Länge eines hülsenförmigen Abschnitts des Abtriebsglieds 7. Insbesondere ist der Arm 10b einschließlich Sperrglied 10a und hülsenförmigem Abschnitt länger als der hülsenförmige Abschnitt des Abtriebsglieds 7. Innerhalb des hülsenförmigen Abschnitts des Abtriebsglieds 7 ist das Antriebsglied 8 in der Gestalt einer vorgespannten wendelförmigen Druckfeder aufgenommen. Das Federelement 8 stützt sich mit seinem proximalen Ende an dem Halteelement 10 und mit seinem distalen Ende an dem distalen Ende des hülsenförmigen Abschnitts des Abtriebsglieds 7, der zugleich die Schulter 7b bildet, ab. Von dem distalen Ende des hülsenförmigen Abschnitts des Abtriebsglieds 7 ragen zwei sich gabelförmig aufweitende Arme ab, die an ihrem distalen Ende jeweils ein Kontaktelement 7a bilden. Die Kontaktelemente 7a weisen abgeschrägte Flächen auf, die sich in Längsrichtung in der Flucht mit der Gehäusewandung des Reservoirteils 5a des Produktbehältnisses 5 befinden. Dies bewirkt, dass die abgeschrägten Flächen der Kontaktelemente 7a bei einer Bewegung des Abtriebsglieds 7 in distale Richtung in einen Anschlag mit dem proximalen Ende des Produktbehältnisses 5 geraten.

Im Folgenden wird die Funktion der Injektionsvorrichtung beschrieben. Ausgehend von dem in den Figuren 3a und 3b gezeigten Auslieferungszustand der Injektionsvorrichtung umgreift der Verwender der Vorrichtung das Gehäuse 1 mit der einen Hand und mit der anderen Hand das Abziehelement 3. Zum Abziehen des Abziehelements 3 zieht der Verwender an dem Abziehelement 3, wodurch dieses vom Gehäuse 1 entfernt wird. Beim Entfernen des Abziehelements 3 sind oder gelangen die Eingriffsglieder 3d in die Lücke zwischen Nadelschutzkappe 6 und Schulter 5b und schließlich in einen Anschlag mit der proximalen Stirnseite der Nadelschutzkappe 6. Ferner werden die Eingriffsglieder 3a beim Abziehen des Abziehelements 3 aus den Ausnehmungen 4f bewegt, verbleiben jedoch in den Nuten 12f.

Die Eingriffsglieder 3a, 3d werden schließlich durch die Innenseite des Produktbehältnishalters 4c an einer Bewegung radial nach außen gehindert, wobei die Eingriffs glieder 3d sogar in den Eingriff in die Lücke bzw. in die Stirnseite der Nadelschutzkappe gezwängt werden. Hierzu ist das radial nach außen Eingriffsglied 3a von Vorteil, da es eine Getriebefläche bildet, die bei der Bewegung des Eingriffsglieds 3a aus der Aussparung 4f an dem Produktbehältnishalter 4 abgleitet und somit mit Hilfe des durch die Innenseite des Produktbehältnishalters 4 gebildeten Halteabschnitts einen Eingriff des Eingriffsglieds 3d in die Nadelschutzkappe 6 sicherstellt. Beim Fortsetzen der Abziehbewegung des Abziehelements 3 nehmen die Eingriffsglieder 3d die Nadelschutzkappe 6 mit, wodurch diese vom Produktbehältnis 5 abgezogen wird, und rastet das Eingriffsglied 3a aus der Lasche 12e oder der Nut 12f aus, da die Stützwirkung für das Eingriffsglied 3a nach innen weggefallen ist. Die mit der Abziehbewegung in das Produktbehältnis eingebrachte äußere Kraft kann von den Schultern 5b des Produktbehältnisses 5 über die verformten Zungen 12a auf den Produktbehältnishalter 4 und vom Produktbehältnishalter 4 über den Eingriff 4b, 1a in das Gehäuse 1 abgeleitet werden. Alternativ oder zusätzlich kann die äußere Kraft von Fingerflansch 5d auf den Produktbehältnishalter und von dort über den Eingriff 4b in das Gehäuse 1 abgeleitet werden. Die Abragung 5d des Produktbehältnisses 5 bleibt bei der ersten Alternative unbelastet, wodurch eine Beschädigung des Produktbehältnisses 5 vermieden wird. Beim Fortsetzen der Abziehbewegung des Abziehelements 3 greift das aus der Nut 12f ausgerastete Eingriffsglied 3a an die ringförmige Basis 11c an und nimmt das Reduzierungsstück 11 mit. Durch das Abziehen des Abziehelements 3 wird auch das Eingriffsglied 2a der Nadelschutzhülse 2 für eine Bewegung nach innen freigegeben, da beim Abziehen auch der Halteabschnitt 3c entfernt wurde.
Durch den Eingriff des Eingriffsglieds 3d in die Nadelschutzkappe 6 wird zusätzlich sichergestellt, dass das Eingriffsglied 3a in den Nuten 12f verbleibt. Bei der Abziehbewegung des Abziehelements 3 stößt das Eingriffsglied 3a an das distale Ende der Nut 12f an. Das Halteglied 12 wird vom Abziehelement 3 mitgenommen. Durch den Anschlag des Rastelements 11e an das Halteglied 12 wird auch das Reduzierungsstück 11 aus seiner ersten Position, die es im fertig montierten Zustand einnimmt, mitgenommen. Die Zunge 12a wird bei der Mitnahme oder Bewegung des Halteglieds 12 in distale Richtung vom Ablenkmittel 4a zur Längsachse hin abgelenkt bzw. verformt. Die verformte Zunge 12a befindet sich dann vor der Schulter 5b, insbesondere zwischen Nadelschutzkappe 6 und Schulter 5b. Die verformte Zunge 12a verrastet mit dem Rastglied 12b, das von der Zunge 12a radial nach außen abragt, mit dem Ablenkmittel 4a oder mit dem Produktbehältnishalter 4. Das Halteglied 12 ist somit axial fest relativ zum Produktbehältnishalter 4.

Das Reduzierungsstück 11, das von dem Abziehelement 3, insbesondere mit Hilfe des Halteglieds 12 und/oder des Eingriffsglieds 3a mitgenommen wird, verrastet mit seinen Rastelementen 11e und 11f axialfest mit der Nadelschutzhülse 2, wie z. B. in den Figuren 4a und 4b zu sehen ist.

In den Figuren 4a und 4b wird die Injektionsvorrichtung von dem das Abziehelement 3 abgezogen wurde, dargestellt. Die Vorrichtung ist nun bereit für die Verwendung. Hierzu umgreift der Verwender der Vorrichtung das Gehäuse 1 und drückt die Injektionsvorrichtung mit ihrem distalen Ende, das von der Nadelschutzhülse 2 gebildet wird, an die Injektionsstelle an. Hierdurch gleitet das Eingriffsglied 2a aus dem Eingriff mit dem Befestigungsgegenglied 1b des Gehäuses 1, so dass das Eingriffsglied 2a nach innen ausgelenkt wird. Zwischen der Nadelschutzhülse 2 und der restlichen Injektionsvorrichtung findet eine Relativbewegung statt, bei der die Nadelschutzhülse 2 über eine rampenförmig ausgestaltete Getriebefläche 4e des Produktbehältnishalters 4 geschoben wird, wodurch der Eingriff des Produktbehältnisses 4 mit der Abragung 1a des Gehäuses 1 gelöst wird, indem das Befestigungsglied 4b nach innen ausgelenkt wird. Ferner werden beim Verschieben der Nadelschutzhülse 2 das Federelement 9 um den Verschiebeweg gespannt und eine von der Nadelschutzhülse 2 gebildete Aussparung 2d axial auf Höhe des Sperrglieds 10a gebracht. Durch das Einschieben der Nadelschutzhülse 2 wird die Einstechsequenz und im weiteren Sinne auch die Ausschüttsequenz ausgelöst. Die Nadelschutzhülse 2 kann somit auch als Auslöseelement 2 bezeichnet werden.

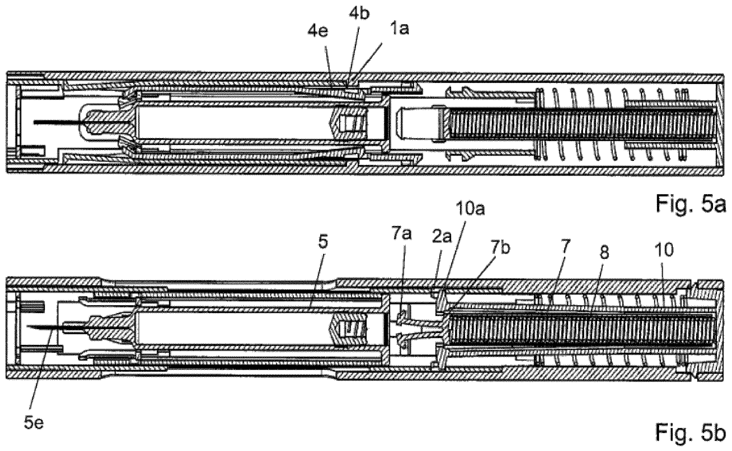

In den Figuren 5a und 5b wird die Injektionsvorrichtung in einem Zustand gezeigt, in dem die Nadelschutzhülse 2 zur Auslösung eingeschoben wurde. Das Befestigungsglied 4b ist aus dem Eingriff mit dem Eingriffsgegenglied 1a, wodurch es für eine Axialbewegung relativ zum Gehäuse 1 freigegeben ist.

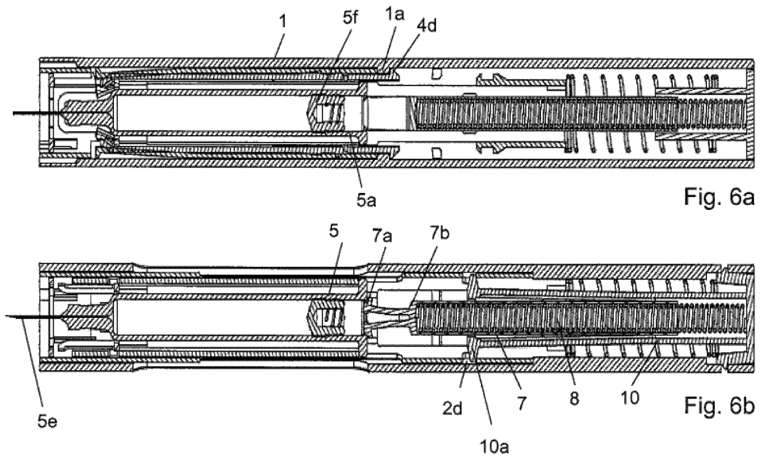

Das Sperrglied 10a kann nun in die Aussparung 2d ausgelenkt werden, wobei es im Eingriff mit der Nadelschutzhülse 2 eine Bewegung der Nadelschutzhülse 2 in distale Richtung sperrt. Gleichzeitig mit der Bewegung in die Aussparung 2d gibt das Sperrglied 10a die Schulter 7b frei, so dass das vorgespannte Antriebsglied 8 das Abtriebsglied 7 in distale Richtung bewegen kann. Der nachfolgend beschriebene Teil einer Gesamtbewegung wird als Einstechsequenz bezeichnet. Bei dieser Bewegung geraten die Kontaktelemente 7a in einen Anschlag mit dem proximalen Ende des Produktbehältnisses 5, wodurch dieses in distale Richtung verschoben wird, und zwar so weit, bis die Nadel 5e durch Öffnung 11d hindurch der gewünschten Einstechtiefe entsprechend weit über das distale Ende der Injektionsvorrichtung hervortritt, wie es in den Figuren 6a und 6b gezeigt wird. Sobald die Nadel 5e um das entsprechende Maß aus dem distalen Ende der Injektionsvorrichtung hervortritt, schlägt ein zweites durch den Produktbehältnishalter 4 gebildetes Befestigungsglied 4d an der Abragung 1a des Gehäuses 1 an, wodurch die Vortriebsbewegung der Nadel 5e gestoppt wird. Die Einstechsequenz ist dadurch beendet.

Durch die abgeschrägten Flächen der Kontaktelemente 7a und der weiterhin wirkenden Kraft des Antriebsglieds 8 gleiten die Kontaktelemente 7a an dem proximalen Ende des Produktbehältnisses 5 ab, so dass sie nach innen, insbesondere aufeinander zu und/oder in den Reservoirabschnitt 5a ausgelenkt werden und somit in einen Anschlag mit dem Kolben 5f gelangen. Hierdurch wird die Ausschüttsequenz gestartet, denn die Kraft des Antriebsglieds 8 verschiebt den Kolben 5f in Richtung Nadel 5e, so dass das im Produktbehältnis 5 enthaltene Produkt über die Nadel 5e ausgeschüttet wird.

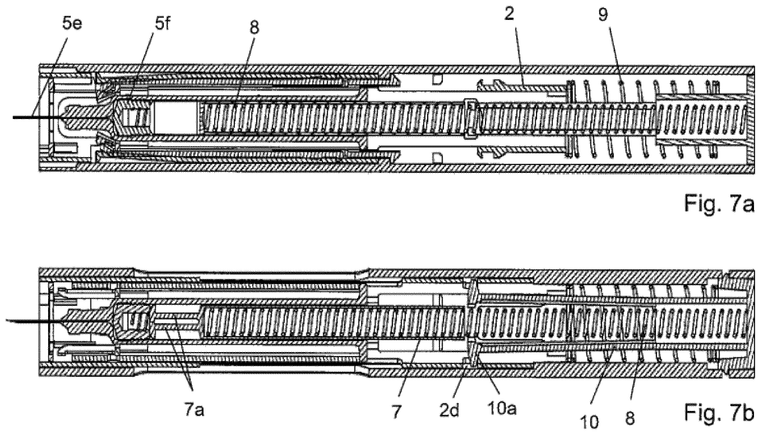

Die Figuren 7a und 7b zeigen die Injektionsvorrichtung am Ende der Produktausschüttsequenz, insbesondere mit in den Reservoirteil 5a ausgelenkten Kontaktelementen 7a.

Während der Einstech- und Ausschüttsequenz verhindert der hülsenförmige Abschnitt des Abtriebsglieds 7, dass die Sperrglieder 10a aus dem Eingriff mit den Aussparungen 2d des Auslöseelements geraten. Am Ende der Produktausschüttsequenz können die Sperrglieder 10a aus dem Eingriff mit der Aussparung 2d bewegt werden, da sich das Abtriebsglied 7 komplett an den Sperrgliedern 10a vorbeibewegt hat.

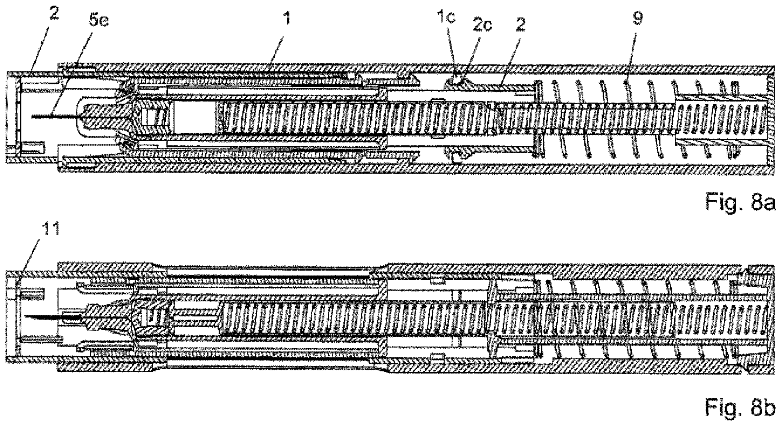

Nachdem der Verwender einige Sekunden nach der beendeten Produktausschüttsequenz die Injektionsvorrichtung von der Injektionsstelle abnimmt, drückt das Federelement 9 die Nadelschutzhülse 2 in distale Richtung, wobei die Sperrglieder 10a aus dem Eingriff mit den Aussparungen 2d bewegt werden. Ferner wird die Nadelschutzhülse 2 mitsamt Reduzierungsstück 11 über das distale Ende der Nadel 5e verschoben, wie es in den Figuren 8a und 8b dargestellt ist.

Um ein Zurückschieben der Nadelschutzhülse 2 ins Gehäuse 1 zu verhindern, weist die Nadelschutzhülse 2 ein Sperrglied 2c auf, welches formschlüssig, vorzugsweise nicht lösbar, d. h. nur durch extreme Kraft bzw. Zerstörung lösbar in die Abragung 1c des Gehäuses 1 eingreift. Ein Zurückschieben der Nadelschutzhülse 2 in das Gehäuse 1 ist somit unter normalen Umständen nicht mehr möglich. Das Hineinstecken z. B. eines Fingers in das distale Ende der Injektionsvorrichtung ist aufgrund des durch das Reduzierungsstück verringerten Querschnitts im Öffnungsbereich nicht möglich. Die Verletzungsgefahr, die von der benutzten Vorrichtung ausgeht, wird somit verringert.

## Patentansprüche

1. Injektionsvorrichtung zur Verabreichung eines Produkts umfassend:
a) ein hülsenförmiges Gehäuse (1),
b) eine Nadelschutzhülse, die in dem Gehäuse längsverschiebbar aufgenommen ist,
c) einen Produktbehältnishalter (4), der ein Produktbehältnis (5) aufnimmt,
d) ein Abtriebsglied (7) mit einem hülsenförmigen Abschnitt, welches auf das Produktbehältnis wirkt,
e) ein Antriebsglied (8), welches Energie liefert,
f) ein Auslöseelement, womit das Antriebsglied (8) freigegeben werden kann,
g) ein Halteelement (10), welches das Antriebsglied (8) bis zur Auslösung der Injektionsvorrichtung in einem gespannten Zustand hält und federnde Arme (10b) aufweist, die sich in Längsrichtung der Injektionsvorrichtung erstrecken und an ihren distalen Enden ein Sperrglied (10a) bilden,
h) **dadurch gekennzeichnet, dass** während einer Ausschüttsequenz der hülsenförmige Abschnitt des Abtriebsgliedes (7) verhindert dass das Sperrglied (10a) aus einem Eingriff mit einer Aussparung (2d) des Auslöseelementes geraten kann, in welche das Sperrglied (10a) ausgelenkt wurde nachdem die Nadelschutzhülse zur Auslösung eingeschoben wurde.

2. Injektionsvorrichtung nach Anspruch 1, **dadurch gekennzeichnet, dass** das Antriebsglied (8) eine vorgespannte Druckfeder ist und innerhalb des hülsenförmigen Abschnitts des Abtriebsglieds (7) aufgenommen ist.

3. Injektionsvorrichtung nach einem der vorhergehenden Ansprüche, **dadurch gekennzeichnet, dass** das Sperrglied (10a) in Bezug auf den Arm (10b) eine nach innen gerichtete und eine nach aussen gerichtete Abragung bildet.

4. Injektionsvorrichtung nach einem der vorhergehenden Ansprüche, **dadurch gekennzeichnet, dass** das Sperrglied (10a) quer zu einer Längsachse (L) der Injektionsvorrichtung bewegbar ist.

5. Injektionsvorrichtung nach Anspruch 3, **dadurch gekennzeichnet, dass** die nach innen gerichtete Abragung an eine von dem Abtriebsglied (7) gebildete Schulter (7b) formschlüssig eingreift, und dadurch verhindert, dass sich das Abtriebsglied (7) in distale Richtung bewegt.

6. Injektionsvorrichtung nach einem der vorhergehenden Ansprüche, **dadurch gekennzeichnet, dass** das Antriebsglied (8) sich mit seinem proximalen Ende an dem Halteelement (10) und mit seinem distalen Ende an einem distalen Ende des hülsenförmigen Abschnitts des Abtriebsglieds (7) abstützt.

7. Injektionsvorrichtung nach einem der vorhergehenden Ansprüche, **dadurch gekennzeichnet, dass** das Halteelement (10) ein proximales Ende des Gehäuses (1) verschliesst.

8. Injektionsvorrichtung nach einem der vorhergehenden Ansprüche, **dadurch gekennzeichnet, dass** ein Federelement (9) Energie für eine Verschiebung der Nadelschutzhülse in eine Nadelschutzposition liefert.

## Claims

1. An injection device for administering a product, comprising:
a) a sleeve shaped housing (1),
b) a needle protecting sleeve accommodated in the housing and displaceable longitudinally,
c) a product container holder (4) accommodating a product container (5),
d) a driven member (7) comprising a sleeve-shaped portion and acting on the product container;
e) a drive member (8) supplying energy;
f) a triggering element with which the drive member (8) may be released,
g) a holding element (10) that keeps the drive member (8) tensed until the injection device is triggered; and that comprises resilient arms (10b) extending in the longitudinal direction of the injection device and forming a blocking member (10a) at their distal ends,
h) **characterized in that**, during an injection sequence, the sleeve-shaped portion of the driven member (7) prevents the blocking member (10a) from passing out of engagement with a cavity (2d) of the triggering element, into which the blocking member (10a) has been deflected after the needle protecting sleeve has been inserted for triggering.

2. The injection device according to claim 1, **characterized in that** the drive member (8) is a pre-strained pressure spring accommodated within the sleeve shaped portion of the driven member (7).

3. The injection device according to one of the preceding claims, **characterized in that** the blocking member (10a) comprises an inwardly directed projection and an outwardly directed projection in relation to the arm (10b).

4. The injection device according to one of the preceding claims, **characterized in that** the blocking member (10a) is movable transverse to a longitudinal axis (L) of the injection device.

5. The injection device according to claim 3, **characterized in that** the inwardly directed projection engages a shoulder (7b) of the driven member (7) in form-fit manner to prevent the driven member (7) from moving in the distal direction.

6. The injection device according to one of the preceding claims, **characterized in that** the proximal end of the drive member (8) is supported on the holding element (10) and the distal end of the drive member (8) is supported on the distal end of the sleeve-shaped portion of the driven member (7).

7. The injection device according to one of the preceding claims, **characterized in that** the holding element (10) seals a proximal end of the housing (1).

8. The injection device according to one of the preceding claims, **characterized in that** a spring element (9) supplies energy for a displacement of the needle protecting sleeve into a needle protecting position.

## Revendications

1. Dispositif d'injection pour l'administration d'un produit, comprenant :
a) un boîtier (1) en forme de manchon,
b) un manchon de protection d'aiguille qui est logé de manière coulissante longitudinalement dans le boîtier,
c) un support de récipient de produit (4), qui loge un récipient de produit (5),
d) un organe de sortie (7) avec une portion en forme de manchon, qui agit que le récipient de produit,
e) un organe d'entraînement (8) qui délivre de l'énergie,
f) un organe de déclenchement, avec lequel l'élément d'entraînement (8) peut être libéré,
g) un élément de maintien (10), qui maintient l'organe d'entraînement (8) jusqu'au déclenchement du dispositif d'injection dans un état serré et qui comprend des bras élastiques (10b), qui s'étendent dans la direction longitudinale du dispositif d'injection et qui forment, au niveau de leurs extrémités distales, un organe de blocage (10a),
h) **caractérisé en ce que**, pendant une séquence de distribution, la portion en forme de manchon de l'organe de sortie (7) empêche que l'organe de blocage (10a) puisse être mis hors de l'emboîtement avec un évidement (2d) de l'élément de déclenchement, dans lequel l'organe de blocage (10a) a été dévié après que le manchon de protection d'aiguille ait été poussé pour le déclenchement.

2. Dispositif d'injection selon la revendication 1, **caractérisé en ce que** l'organe d'entraînement (8) est un ressort de compression précontraint et est logé à l'intérieur de la portion en forme de manchon de l'organe de sortie (7).

3. Dispositif d'injection selon l'une des revendications précédentes, **caractérisé en ce que** l'organe de blocage (10a) forme, par rapport au bras (10b), une saillie orientée vers l'intérieur et une saillie orientée vers l'extérieur.

4. Dispositif d'injection selon l'une des revendications précédentes, **caractérisé en ce que** l'organe de blocage (10a) est mobile transversalement par rapport à un axe longitudinal (L) du dispositif d'injection.

5. Dispositif d'injection selon la revendication 3, **caractérisé en ce que** la saillie orientée vers l'intérieur s'emboîte par complémentarité de forme avec un épaulement (7b) formé par l'organe de sortie (7) et empêche ainsi que l'organe de sortie (7) se déplace dans la direction distale.

6. Dispositif d'injection selon l'une des revendications précédentes, **caractérisé en ce que** l'organe d'entraînement (8) s'appuie, avec son extrémité proximale, contre et l'élément de maintien (8) et, avec son extrémité distale, contre une extrémité distale de la portion en forme de manchon de l'organe de sortie (7).

7. Dispositif d'injection selon l'une des revendications précédentes, **caractérisé en ce que** l'élément de maintien (10) obture une extrémité proximale du boîtier (1).

8. Dispositif d'injection selon l'une des revendications précédentes, **caractérisé en ce qu'**un élément à ressort (9) délivre de l'énergie pour pour une poussée du manchon de protection d'aiguille vers une position de protection d'aiguille.
